# EUROPEAN PATENT APPLICATION

(11) **EP 0 566 359 A2**
(43) Date of publication of application: **20.10.1993**
(21) Application number: 93302849.0
(22) Date of filing: 13.04.1993
(51) Int. Cl.: A61B 17/34

(54) **Illuminated surgical cannula**

(30) Priority: 14.04.1992 US 868107
(71) Applicant: ETHICON INC., Somerville New Jersey 08876 (US)
(72) Inventor: Shobert, James P., South Bend, Indiana 46614 (US)
(74) Representative: Fisher, Adrian John

(57) **Abstract**

A surgical trocar is disclosed which includes a fiber reinforced plastic cannula having longitudinally extending optical grade fibers which transmit light to the end of the cannula to serve to illuminate the surgical site.

## Description

### BACKGROUND OF THE INVENTION

This invention relates to surgical instruments and, more particularly, to an improved cannula for instruments used in laproscopic surgery.

Laproscopic surgery techniques involve performing surgical procedures through a small tube that is inserted through a small incision in the patient. These techniques minimize trauma to surrounding tissue and organs and greatly reduce the recovery period which, in many cases, is due to the size of the incision required to gain access to the surgical site. To perform these laproscopic techniques, a small incision is made, and a small tube or cannula is inserted through the incision. A trocar having a sharpened point is carried by the cannula and, in the case of abdominal surgery, insufflating gas may be passed through the cannula to inflate the abdominal cavity. The cannula comprises a port through which various instruments can be inserted to perform procedures, such as cutting, suturing and removal of organs, such as the gall bladder. These procedures involve far less trauma in the patient, less recovery time, and the ability to control hospital costs by the elimination of large incisions on the patient.

The cannula employed to perform these procedures is made from stainless steel tubing. Among the problems associated with stainless steel are its thermal and electrical conductivity, and its tendency to reflect and scatter the X-rays associated with scanning devices.

A further problem involved in the use of prior art cannulae is the need for illumination at the surgical site. This is presently accomplished by employing fiber optics, which are either inserted through the cannula to occupy valuable space within the cannula, or are inserted through a separate incision in the patient.

### BRIEF DESCRIPTION OF THE INVENTION

This invention overcomes the problem of providing illumination at the operation site and the problems associated with the use of stainless steel tubing for the cannula.

According to this invention, a cannula for laproscopic surgery comprises a thin walled plastic tube having a cylindrical sidewall reinforced with continuous filaments embedded in the plastic for imparting both torsional and flexural strength thereto which compares favorably with the physical properties of stainless steel, at least on a strength-to-weight basis. The reinforcement comprises a plurality of filaments which, preferably, are glass filaments extending in one circumferential direction in the form of a helix alternately over and under successive axially extending continuous filaments. Other filaments extend in an opposite circumferential direction in the form of a helix alternately over and under the axially extending continuous filaments and alternately over and under the successive filaments of the first mentioned helix. At least some of the axially extending filaments are fiber optic filaments adapted to transmit light from one end of the tube to the other from a fiber optic connector terminal associated with a source of artificial light.

The technique for forming tubes which may be used as cannulas, according to this invention, may be found in U.S. Patent No. 3,007,497, to Samuel M. Shobert, the subject matter of which is incorporated herein by reference.

### BRIEF DESCRIPTION OF THE DRAWINGS

FIG. 1 is an elevational view, partly in section, of a surgical trocar embodying the present invention;
FIG. 2 is a diagrammatic illustration, partly in section, of an apparatus employed in fabricating the cannula of this invention;
FIG. 3 is a top plan view, in diagrammatic form, of the apparatus illustrated in FIG. 2;
FIG. 4 is a cross-sectional view of the cannula; and
FIG. 5 is an enlarged view of a small area of the fibrous reinforcement of the sidewall of the cannula.

### DETAILED DESCRIPTION OF THE DRAWINGS

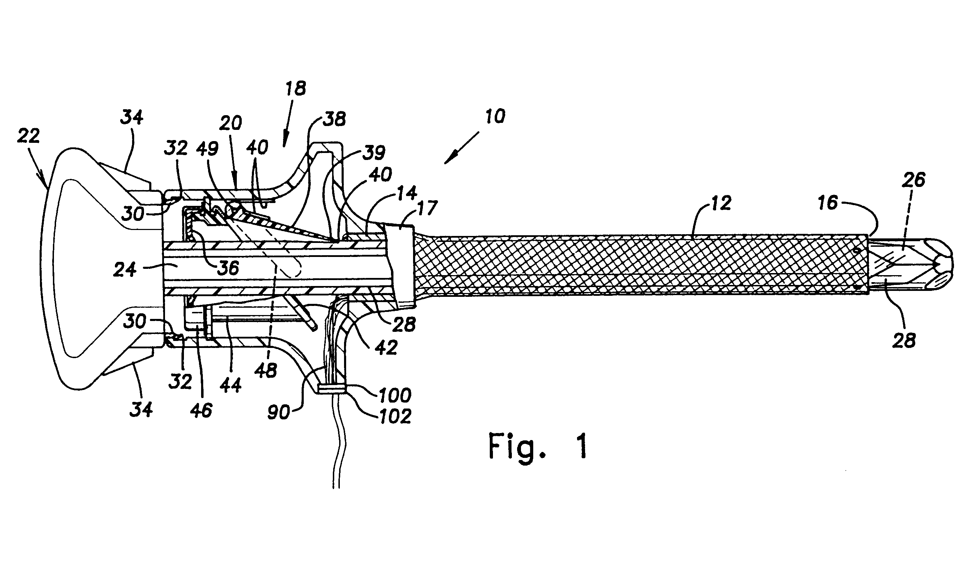

Referring now to the drawings and, particularly, to FIG. 1, there is illustrated a surgical trocar which is employed in laproscopic surgical techniques. The trocar 10 includes a cannula 12 which comprises a thin walled plastic tube reinforced with filaments embedded therein. The cannula has proximal and distal ends 14 and 16, respectively, and is mounted in a hub portion 17 of a housing 18. The housing 18 includes a body portion 20 and a removable handle portion 22.

As is set forth in more detail in U.S. Patent No. 4,654,030, the subject matter of which is incorporated herein by reference, the handle portion 22 has an obturator 24 affixed thereto, and the obturator 24 has a piercing tip 26 at its distal end. Further, as is described in the aforementioned patent, the obturator 24 is shielded by a generally tubular, clear plastic obturator shield or sleeve 28 which is spring biased toward the distal end of the obturator by a biasing mechanism (not shown) contained in the handle portion 22. Thus, the shield 28 normally covers the obturator tip during storage of the handle portion 22 and during the assembly operation.

To assemble the device, the handle portion 22 is axially aligned with the body portion 20, and the obturator 24 and its surrounding shield 28 are inserted through the cannula until spring biased clips 30 engage recesses 32 in the body portion 20. The clips 30 have body portions 34 which project from the handle portion 22 so that the clips 30 may be disengaged by pressing the body portions 34 inwardly. The biasing mechanism in the handle portion 22 ensures that the shield 28 remains in its normally extended position as the obturator passes through an apertured diaphragm seal 36 and a spring biased flap valve 38. An end 39 of the flap valve engages a notch 40 in the sheath 28 to ensure against inadvertent retraction of the shield 28.

The flap valve 38 is normally biased by a wire spring 40 so that the valve 38 normally seats against a sealing ring 42 of a tubular portal assembly 44 when the obturator 24 and its shield 28 are removed from the body portion 20. The assembly 44 also carries the diaphragm 36 which is mounted on the portal member 44 by a crimped band 46.

The trocar 10 is inserted through the wall of a body cavity while the valve 38 is manually held out of engagement with the recess 40 by operating a lever 48 which is fixed to a pivot pin 49 associated with the valve 38 and located on an opposite face of the body portion 20. Engagement of the skin of the patient by the distal end of the shield 28 causes the shield to retract against the bias of the spring mechanism and permits the obturator tip 26 to pierce the cavity wall. After the cavity wall has been pierced in this manner, the sheath 28 is automatically extended by the spring and is locked in place by engagement of the end 39 of the flap valve 38 in the notch 40 to shield the tip 26 from inadvertently piercing the patient. With the cannula 12 in its intended location, the handle portion 22 and its associated obturator 24 and sheath h 28 may be separated from the body portion 20 of the trocar 18 by pressing the body portions 34 of the clips 30 and releasing the end 39 from the notch 40 by operating the lever48. After retraction of these members, the flap valve 38 closes to prevent egress of any insufflating gas which may have been pumped into the body cavity.

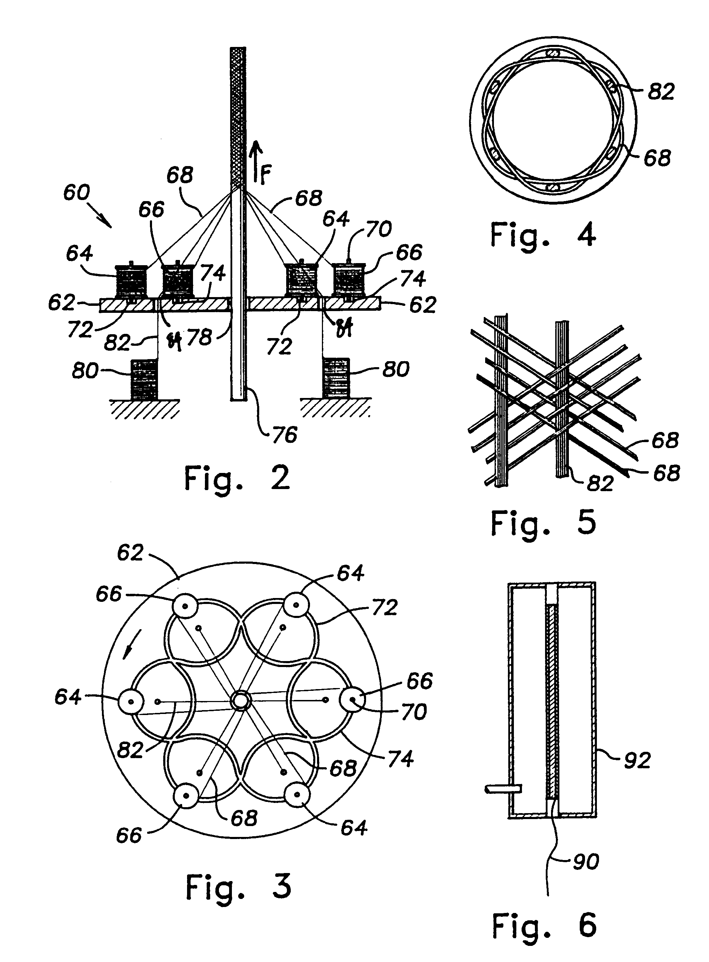

As was previously mentioned, the cannula 12 is made from a fiber reinforced plastic material having axially extending fiber optic filaments adapted to transmit lightfrom one end of the cannula to the other. Referring now to FIGS. 2-5, there is illustrated a technique for fabricating the cannula 12. A conventional braiding machine 60 has a supporting table 62 which carries a number of roving cakes 64 and 66. The roving cakes 64 and 66 comprise glass filaments 68 and are supported on spindles 70 which are movable in undulating tracks 72 and 74.

A mandrel 76 extends through a clearance opening 78 in the table 62.

Beneath the table 62 are disposed a plurality of roving cakes 80. At least some of the roving cakes 80 contain optical quality filaments 82 suitable for the axial transmission of artificial light therethrough and suitable for additional or complete longitudinal reinforcement of the cannula 12 depending upon the presence or absence of normal glass reinforcement fibers provided by one or more of the roving cakes 80. The filaments 82 are trained through openings 84 in the table 62 and, together with the filaments 68, are affixed to the mandrel 76 by adhesive tape or the like. With the filaments thus fixed to the mandrel, the mandrel is raised at a constant rate in the direction of the arrow F while the roving cakes 64 and 66 are rotated in opposite directions in the tracks 72 and 74. Since the roving cakes 64 and 66 travel in their undulating paths, they cause the filaments to pass over and under adjacent filaments and over and under the filaments 82 which are captured by the helically wound filaments and extend in an axial direction with respect to the mandrel 76. This weaving pattern is illustrated in FIGS. 4 and 5.

After the proper length of winding is achieved on the mandrel 76, the optical fibers 82 are bundled in a suitable sheath 90, the filaments 68 are trimmed, and the mandrel and the filaments are immersed or wetted in a bath of liquid resin material, such as epoxy resin. The mandrel and the wetted fibers are then placed in a steam jacketed mold 92, and the resin is cured by heat. After the resin is cured, the mandrel is removed from the mold; and the mandrel is then removed from the completed cannula 12.

When the cannula 12 is mounted in the hub portion 16 of the housing 20, the optical fibers 82 in the sheath 90 are terminated in a suitable connector 100 (FIG. 1), and the connector 100 cooperates with a mating connector 102 which leads to a source of artificial light.

During surgical procedures, therefore, the cannula may be illuminated at its distal end to provide light at the site of the surgical procedure.

Although the preferred embodiments of this invention have been shown and described, it should be understood that various modifications and rearrangements of the parts may be resorted to without departing from the scope of the invention as disclosed and claimed herein.

## Claims

1. A cannula for laproscopic surgery comprising a thin-walled plastic tube, said tube having at least one longitudinally extending fiber optic filament adapted to transmit light from one end of the tube to the other, and means for connecting said fiber optic filament to a source of artificial light.

2. A cannula according to claim 1, wherein at least said axially extending filaments are glass.

3. A cannula for laproscopic surgery, comprising a thin-walled plastic tube, said tube having a cylindrical sidewall reinforced with continuous filaments embedded in said plastic for imparting both torsional and flexural strength thereto, said reinforcement comprising a plurality of filaments extending in one circumferential direction in the form of a helix alternately over and under successive axially extending continuous filaments, other filaments extending in an opposite circumferential direction in the form of a helix alternately over and under said axially extending continuous filaments and alternately over and under the successive filaments of the first mentioned helix, said axially extending filaments being fiber optic filaments adapted to transmit light from one end of said tube to the other, and means for connecting said fiber optic filaments to a source of artificial light.

4. A cannula according to claim 3, wherein at least said axially extending filaments are glass.

5. In a trocar assembly comprising an elongated trocar obturator having a piercing tip at its distal end, mounting means for said trocar comprising a handle and a cannula attached to said handle for respectively detachably mounting the proximal end of said trocar and providing a guide path to a surgical site, in combination therewith the improvement wherein said cannula comprises a thin-walled plastic tube, said tube having a cylindrical sidewall reinforced with filaments embedded in said plastic for imparting both torsional and flexural strength thereto, said filaments including a plurality of axially extending filaments, said axially extending filaments being fiber optic filaments adapted to transmit light from one end of said tube to the other, and means for connecting said fiber optic filaments to a source of artificial light.

6. A trocar assembly according to claim 5, wherein at least said axially extending filaments are glass.

7. In a trocar assembly comprising an elongate trocar obturator having a piercing tip at its distal end, mounting means for said trocar comprising a handle and a cannula attached to said handle for respectively detachably mounting the proximal end of said trocar and providing a guide path to a surgical site, in combination therewith the improvement wherein said cannula comprises a thin walled plastic tube, said tube having a cylindrical sidewall reinforced with continuous filaments embedded in said plastic for imparting both torsional and flexural strength thereto, said reinforcement comprising a plurality of filaments extending in one circumferential direction in the form of a helix alternately over and under successive axially extending continuous filaments, otherfilaments extending in an opposite circumferential direction in the form of a helix alternately over and under said axially extending continuous filaments and alternately over and under the successive filaments of the first mentioned helix, said axially extending filaments being fiber optic filaments adapted to transmit light from one end of said tube to the other, and means for connecting said fiber optic filaments to a source of artificial light.

8. A trocar assembly according to claim 6, wherein at least said axially extending filaments are glass.
